(19) Europäisches Patentamt
European Patent Office
Office européen des brevets

(11) Numéro de publication: **0 068 566**
**B1**

(12) # FASCICULE DE BREVET EUROPEEN

(45) Date de publication du fascicule du brevet: **21.11.84**

(51) Int. Cl.³: **A 61 K 9/20**, A 21 D 2/08

(21) Numéro de dépôt: **82200750.6**

(22) Date de dépôt: **17.06.82**

(54) **Forme médicamenteuse d'une composition de matières comestible contenant un diméthylpolysiloxane fluide.**

(30) Priorité: **30.06.81 BE 205270**

(43) Date de publication de la demande: **05.01.83 Bulletin 83/1**

(45) Mention de la délivrance du brevet: **21.11.84 Bulletin 84/47**

(84) Etats contractants désignés: **AT BE CH DE FR GB IT LI LU NL SE**

(56) *Documents cités:*
**EP - A - 0 009 913**
**BE - A - 889 453**
**DE - A - 1 467 745**
**GB - A - 2 033 915**
**US - A - 3 422 189**
**US - A - 4 127 650**

**CHEMICAL ABSTRACTS, vol.95, no.4, 27 juillet 1981, page 342, résumé no.30432w, Columbus, Ohio (US)**

(73) Titulaire: **Sociéte d'Importation de Parfumerie et de Produits Pharmaceutiques en abrégé "Sipar-Pharma" Société Anomyme, rue de l'Orme, 42, B-1040 Bruxelles (BE)**

(72) Inventeur: **Rousseau, Jean Augustin Louis, Rue Nicaise 6, B-1341 Ceroux-Mousty (BE)**

(74) Mandataire: **De Brabanter, Maurice et al, Bureau VANDER HAEGHEN 63 Avenue de la Toison d'Or, B-1060 Bruxelles (BE)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 068 566 B1

BUNDESDRUCKEREI BERLIN

## Description

La présente invention est relative à une forme médicamenteuse d'une composition de matières comestible contenant comme ingrédient actif, un diméthylpoysiloxane fluide et éventuellement de la silice finement divisée.

On connaît par le brevet britannique No. 2.033.915 des compositions de matières comestibles, notamment sous forme de comprimés, de capsules, de gels ou de suspensions, contenant un mélange de diméthylpolysiloxane fluide et de silice finement divisée, encore dénommé diméthicone activée ou siméthicone, ayant des propriétés lubrifiantes et anti-moussantes, destinées à prévenir et à atténuer les conséquences des perturbations du processus d'assimilation alimentaire qui entraînent une distension gazeuse, un ballonnement et/ou des lourdeurs du système digestif. Elles favorisent l'élimination des gaz et protègent les muqueuses ou parois digestives, en particulier celles de l'oesophage, de l'estomac et des intestins en les couvrant d'une fine couche de substance active de la composition susdite.

Les compositions de matières connues présentent cependant divers inconvénients, notamment un caractère organoleptique désagréable et un goût insipide, qui n'incite pas à leur consommation et ne favorise pas la salivation.

Leur aspect médicamenteux peut être un obstacle psychologique à leur prise régulière, notamment dans des lieux publics tels que des restaurants.

La quantité de sucre à incorporer dans les comprimés en vue d'obtenir l'édulcoration des ingrédients actifs peut être une source de contreindications.

Lorsque les compositions de matières connues se présentent sous forme de comprimés à avaler, leurs ingrédients actifs ne sont livérés qu'au niveau de l'estomac ou de l'intestin, en sorte qu'ils n'agissent qu'avec retard et n'exercent que peu ou pas d'effets sur les voies digestives supérieures.

Lorsqu'elles se présentent sous forme de suspension ou de sirop, l'utilisateur éprouve des difficultés pour absorber une dose précise et le traitement ambulatoire est rendu plus difficile. En outre, le sirop est sujet à une fermentation s'il n'est pas suffisamment aseptisé ni suffisamment surconcentré en sucre.

Les compositions liquides sont avalées sans mastication de telle sorte que l'absorption de ces produits ne favorise pas la sécrétion de salive à laquelle les ingrédients actifs doivent avantageusement se mélanger.

La présente invention vise à remédier aux inconvénients décrits ci-dessus et a pour objet un produit comestible du type décrit plus haut que est facile et agréable à absorber à des doses prédéterminées, sans obstacles d'ordre psychologique ou autre.

L'invention concerne une forme médicamenteuse d'une composition de matières solide comestible contenant, comme ingrédient actif, un diméthylpolysiloxane fluide et éventuellement de la silice finement divisée, cette composition étant essentiellement caractérisée en ce qu'elle consiste en un biscuit.

La forme de présentation suivant l'invention, consiste avantageusement en un petit biscuit de forme quelconque, par exemple circulaire, bombée contenant environ 10% en poids de diméthylpolysiloxane activé par 4 à 7% en poids de silice finement divisée.

Dans une forme de présentation particulière de l'invention, l'édulcoration desdites matières est obtenue par incorporation en quantités judicieusement choisies dans un biscuit contenant environ 64% de farine de céréale, environ 12,5% de sucre et environ 10% de matières grasses.

L'exemple suivant décrit la composition d'un biscuit suivant l'invention à titre non limitatif.

### Exemple

Ingrédients (pour 100 g de biscuit)

a) Matières actives

| | |
|---|---|
| Diméthicone activée (siméthicone) | 10,6 g Pharmacopée américaine XX, p. 724 |

b) Excipients

| | |
|---|---|
| Farine (sans humidité) | 64 g |
| Sucre | 12,5 g |
| Oleo Oil (bōeuf fractionné) | 9,4 g |
| Eau | 1,5 g |
| Poudre de lait entier | 1,5 g |
| Sel | 0,3 g |
| Bicarbonate de soude | 0,1 g |
| Ethylvanilline | 0,01 g |

c) Valeurs énergétiques

| | |
|---|---|
| Protides | 6,8 g |
| Lipides | 10,2 g |
| Glucides | 69,9 g pour 100 g de produit fini |
| KJoules | 1668 |
| Kcal | 400 pour 100 g de produit fini |

d) Valeur unitaire

| | |
|---|---|
| Poids net minimum par biscuit | 1,4 g |
| Kcal par biscuit environ | 6 Kcal |

Siméthicone par biscuit: environ 0,148 g

Les biscuits suivant la présente invention sont obtenus par un procédé classique de préparation de biscuits sensiblement secs impliquant la cuisson d'une pâte aqueuse à une température et pendant une durée suffisantes pour obtenir un produit sensiblement sec et croquant sans dégradation des ingrédients actifs.

La siméthicone utilisée dans les compositions de matières comestibles suivant l'invention est constituée d'un mélange de polymères de silicone linéaires complètement méthylés contenant des unités récurrentes de formule

$$[-(CH_3)_2SiO-]_n$$

dans laquelle n est compris entre 200 et 350 stabilisée par des radicaux triméthylsiloxy terminaux de formule

$$[(CH_3)_3SiO-]$$

et de silice finement divisée. La siméthicone ne contient pas moins de 90,5% et pas plus de 99,0% de polydiméthylsiloxane de formule

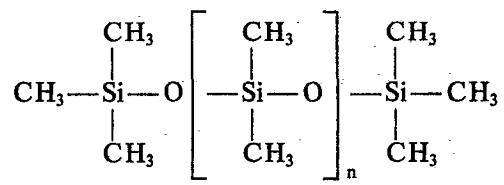

où n est compris entre 200 et 350, tandis que sa teneur en silice est comprise entre 4.0 et 7.0% en poids.

Les compositions de matières sous forme de biscuit suivant l'invention sont, de préférence, absorbées à raison de deux biscuits d'un poids d'environ 1.4 g contenant environ 10% de somethicone, avant chacun des principaux repas ou avant la prise d'une boisson ou d'un aliment susceptible de produire une irritation.

Pour obtenir les meilleurs résultats les produits doivent être mastiqués pendant quelques secondes avant d'être avalés. En cas de nécessité, on peut répéter la prise de deux biscuits après un repas indigeste ou au moment de l'apparition de symptômes de flatulence, distensions gazeuses, lourdeurs, ballonnements ou autres. Les biscuits sont également utilisables à titre préventif dans les cas où une protection des muqueuses du système digestif est souhaitée, pour éviter une irritation due aux alcools, épices, vinaigre, tabac et autres produits agressifs.

Aucun effet secondaire et aucune contreindication ne sont à craindre étant donné que la siméthicone n'est pas assimilée par l'organisme. Ce produit joue exclusivement un rôle physique et est éliminé tel quel. De plus, il n'affecte pas les fonctions enzymatiques digestives, la sécrétion d'acide ni l'assimilation des aliments et des médicaments. Les compositions suivant l'invention peuvent être absorbées par les adultes et les enfants, y compris les femmes enceintes, en raison de leur absence de toxicité, même régulièrement pendant une période prolongée.

La composition de matières suivant l'invention sous forme de petits biscuits est assimilable à des amuse-gueules que l'on absorbe communément avec un apéritif.

Elle se présente avantageusement sous forme de biscuits compacts et pauvres en valeurs caloriques permettant d'absorber facilement une dose assurant une efficacité appréciable, éventuellement emballés individuellement.

On a constaté que l'effet de soulagement des perturbations du système digestif est extrêmement rapide, sinon immédiat lors de l'absorption d'une composition suivant l'invention sous forme de

biscuits. Bien que la demanderesse ne désire pas être liée par une quelconque théorie, elle est d'avis que cette rapidité d'action est due à la salivation accrue qui se manifeste lors de la mastication des biscuits et assure la libération immédiate de la siméthicone et sa répartition uniforme dans la totalité du système digestif.

La siméthicone peut être l'un ou l'autre des produits commerciaux à base de diméthylpolysiloxane.

L'emballage individuel précité peut être un emballage hermétique sous feuille d'aluminium ou un quelconque emballage étanche utilisé en particulier dans l'industrie des médicaments pour conserver un produit à l'abri de l'air et de l'humidité.

## Revendications

1. Forme médicamenteuse d'une composition de matière solide comestible contenant comme ingrédient actif, un diméthylpolysiloxane fluide et éventuellement de la silice devisée, caractérisée en ce qu'elle consiste en un biscuit.
2. Forme médicamenteuse suivant la revendication 1, caractérisée en ce que le biscuit contient environ 10% en poids de diméthylpolysiloxane activé par 4 à 7% en poids de silice finement divisée.
3. Forme médicamenteuse suivant l'une quelconque des revendications 1 et 2, caractérisée en ce que le biscuit contient environ 64% de farine de céréale, environ 12,5% de sucre et environ 10% de matières grasses.
4. Forme médicamenteuse suivant l'une quelconque des revendications précédentes, caractérisée en ce que le biscuit contient un arôme physiologiquement acceptable.
5. Forme médicamenteuse suivant l'une quelconque des revendications précédentes, caractérisée en ce que le biscuit sensiblemsent sec présente un poids d'environ 1.5 gramme.
6. Forme médicamenteuse suivant l'une quelconque des revendications précédentes, caractérisée en ce que le biscuit est emballé hermétiquement et individuellement sous feuille d'aluminium.

## Patentansprüche

1. Eßbare Arzneimittelform einer Stoffzusammensetzung, die als aktiven Bestandteil flüssiges Dimethylpolysiloxan und gegebenenfalls verteiltes Siliziumoxid enthält, dadurch gekennzeichnet, daß sie aus einem Gebäck besteht.
2. Arzneimittelform nach Anspruch 1, dadurch gekennzeichnet, daß das Gebäck etwa 10 Gew.-% Dimethylpolysiloxan enthält, das durch 4—7 Gew.-% feinverteiltes Siliziumoxid aktiviert ist.
3. Arzneimittelform nach Anspruch 1 oder 2, dadurch gekennzeichnet, daß das Gebäck etwa 64% Getreidemehl, etwa 12,5% Zucker und etwa 10% Fett enthält.
4. Arzneimittelform nach einem der Ansprüche 1 bis 3, dadurch gekennzeichnet, daß das Gebäck ein physiologisch verträgliches Aroma enthält.
5. Arzneimittelform nach einem der Ansprüche 1 bis 4, dadurch gekennzeichnet, daß das weitgehend trockene Gebäck ein Gewicht von etwa 1,5 g aufweist.
6. Arzneimittelform nach einem der Ansprüche 1 bis 5, dadurch gekennzeichnet, daß das Gebäck hermetisch dicht und einzeln in Aluminiumfolie eingesiegelt ist.

## Claims

1. A medicinal form of a composition of edible solid matter containing as the active ingredient a fluid dimethyl polysiloxane and possibly devided silica, characterized in that it consists of a biscuit.
2. A medicinal form according to Claim 1, characterized in that the biscuit contains approximately 10% by weight of activated dimethyl polysiloxane to between 4 and 7% by weight of finely divided silica.
3. A medicinal form according to one of Claims 1 and 2, characterized in that the biscuit contains approximately 64% of cereal flour, approximately 12,5% of sugar and approximately 10% of fatty substances.
4. A medicinal form according to any one of the preceding Claims, characterized in that the biscuit contains a physiologically acceptable aroma.
5. A medicinal form according to any one of the preceding Claims, charcterized in that the substantially dry biscuit has a weight of approximately 1.5 grammes.
6. A medicinal form according to any one of the preceding Claims, characterized in that the biscuit is wrapped hermetically and individually in an aluminium foil.